# EUROPEAN PATENT SPECIFICATION

(11) **EP 1 757 234 B1**
(45) Date of publication and mention of the grant of the patent: **14.12.2011**
(21) Application number: 06015228.7
(22) Date of filing: 21.07.2006
(51) Int. Cl.: A61B 17/04

(54) **Extraction device and medical suturing device set**
Extraktionsvorrichtung und Nähset für medizinische Zwecke
Dispositif d'extraction et ensemble de suture destiné à des applications médicales

(30) Priority: 23.08.2005 JP 2005241131
(43) Date of publication of application: 28.02.2007
(73) Proprietor: Covidien AG, 8212 Neuhausen am Rheinfall (CH)
(72) Inventor: Funamura, Shigeaki Nippon Sherwood Med. Ind.Co Ltd, Fukuroi-shi Shizuoka-ken (JP); Mabuchi, Hitoshi Nippon Sherwood Med. Ind.Co Ltd, Fukuroi-shi Shizuoka-ken (JP)
(74) Representative: Tomlinson, Edward James

(56) References cited:
- EP-A1- 0 706 779
- EP-A1- 0 717 957
- EP-A1- 1 598 017
- WO-A1-2004/075761
- US-A- 5 281 237
- US-A- 5 364 410
- US-A- 5 681 333
- US-A- 6 022 360

## Description

The present invention pertains to an extraction device and medical suturing device set used when anchoring human organs and paries.

Generally, when a catheter is inserted transdermally into the stomach, gallbladder, or other organs, a problem may arise in that the organ separates from the paries such that the catheter cannot be inserted into the organ. Therefore, it is necessary to anchor the organ and paries prior to catheter insertion and prevent the organ from separating from the paries.

Recently, the mainstream of medical suturing device sets has become a device in which two needles are used to hand over the suture within an organ, and knot both ends of the suture drawn out of the organism, thus anchoring the organ and paries. Such a device comprises a suture, a suture insertion needle capable of inserting a suture into an organ, and an organ anchoring needle equipped with a tubular external needle and an internal needle having a tip equipped with a snare loop inserted into and capable of sliding within the external needle; by means of the snare loop that bends axially with respect to the internal needle and that protrudes from the tip aperture of the external needle pursuant to the sliding motion of the internal needle, one end of the suture protruding from the suture insertion needle is supported and drawn out from the organism (for example, refer to Japanese Patent Application No. JP 5-161655 (1993), in particular Figure 4). Further suturing devices are shown in US 5,364,410, EP 0 717 957 A1, US 6,022,360, EP 0 706 779 A1, US 5,281,237 and US 5,681,333 wherein a single hook element is described. WO 2004/075761 A1 is a further document showing two suture insertion needles and a single suture grasping needle. The two-part form of claim 1 is based on document EP 0 706 779 A1.

In a conventional medical suturing device set, two needles are used to hand over the suture, so when the relative positions of the needle tips within the organ after puncturing are not correct, or when there is insufficient space within the organ and the snare loop is deformed against the organ wall, it becomes difficult for the suture to be supported by the snare loop, so its use is limited to experienced health care personnel.

### SUMMARY OF THE INVENTION

The object of the present invention is to offer an extraction device and medical suturing device set that solves the aforementioned problems by readily handing over the suture, so that it can be handled even by inexperienced health care personnel.

The invented extraction device is an extraction device used to extract a suture positioned within an organism to the outside of the organism, characterized by the fact that this is furnished with an extraction needle in which an engagement member is formed capable of catching onto that part of the suture positioned circularly within the organism, and drawing this outside of the organism.

In the present invention, the suture inserted into the organism is engaged by the engagement member of the extraction needle, so it can be drawn out of the organism at a place that is different from the insertion location. Because this makes it easy to hand over the suture, it can be handled even by inexperienced health care personnel.

In one invented extraction device, the extraction device is furnished with a mantle that forms a cavity that permits the extraction needle to pass entirely through so as to be exposed from the tip. In the present invention, it is possible to insert the extraction needle into the cavity of the mantle, thus making it possible to reduce the invasiveness to the body when drawing the extraction needle on which the suture is caught outside of the body.

In one invented extraction device, the extraction device is furnished with: an extraction puncture needle formed with an internal cavity from the basal tip side through the apical tip side; and a pulling out device in which is formed an engagement member that can be inserted through the internal cavity so as to be exposed from the apical tip side of the extraction puncture needle, such that the portion that is exposed from the apical tip side of the extraction puncture needle engages part of the suture that has been positioned in a circular shape within the organism and draws it out from the organism.

In the present invention, it is possible to engage the suture that has been inserted into the organism by the engagement member of the extraction device, and draw it out of the organism, though the cavity of the extraction puncture needle, at a location that is different from the insertion location. This makes it easy to hand over the suture, so it can be handled even by inexperienced health care personnel.

In the extraction device of the invention, the engagement member is structured by a series of multiple hook elements; the hook elements are formed with a base that can support part of the suture, a primary extension that is connected to part of the base and extends towards the basal tip of the pulling out device, a secondary extension that is connected to the other part of the base and that extends to the basal tip of the pulling out device, and a gap that is formed so as to envelope the periphery of the suture by the base and the primary extension and the secondary extension, and the series of hook elements is structured so that the primary extension of a hook element is connected to the secondary extension of another hook element that is adjacent to the hook element.

In the present invention, it is possible to have a structure in which the hook elements are facing several different directions outwards, towards 2 outer directions, or towards 4 outer directions; there is no limitation of the peripheral positioning of hook elements, which thus makes it easy to engage the suture that has been disposed in an annular form.

In one invented medical suturing device set, a medical suturing device set that is used to anchor the organ of an organism to the paries of the aforementioned organism is furnished with: an insertion puncture needle, in which is formed an insertion through-hole from the basal tip side through the apical tip side, and that is able to puncture the paries from the outside such that the aperture of the insertion through-hole on the apical tip side is positioned within the organ; a suture that, passing through the insertion through-hole, is supplied within the organ from the aperture, and that is able to form a circular section within the organ; and any one of the aforementioned extraction devices that is able to engage part of the circular section of the suture and draw it out of the organism.

In the present invention, provision is made for an extraction device, an insertion puncture needle, and a suture, which makes it easy to hand over the suture, so the desired organ and paries can be securely anchored with low invasiveness even by inexperienced health care personnel. It is also possible to draw out a single suture from multiple locations, thus, by taking this together and ligating it, it becomes possible to anchor the organ in a plane. Here, anchoring the organ in a plane means that, for example, the organ is anchored by suturing such that the suture forms a triangular or quadrilateral surface within the organ. When the organ is thus anchored, there is no separation within the plane of the organ from the paries.

### BRIEF INTRODUCTION TO THE DRAWINGS

An embodiment of the present invention will now be described by way of example only with reference to the accompanying drawings in which:
Figure 1 is a side view of the medical suturing device set pertaining to an embodiment of the present invention.
Figure 2 is an explanatory diagram of the suture and the insertion puncture needle of Figure 1.
Figure 3 is a side view of the essential elements of the extraction device of Figure 1.
Figure 4 is a diagram explaining the operation of Embodiment 1 of the present invention.
Figure 5 is a diagram explaining the operation subsequent to Figure 4.
Figure 6 is a diagram explaining the operation subsequent to Figure 5.
Figure 7 is a diagram explaining the operation subsequent to Figure 6.
Figure 8 is a diagram explaining the operation subsequent to Figure 7.
Figure 9 is a diagram explaining the operation subsequent to Figure 8.

### DETAILED DESCRIPTION OF THE INVENTION

### Embodiment 1

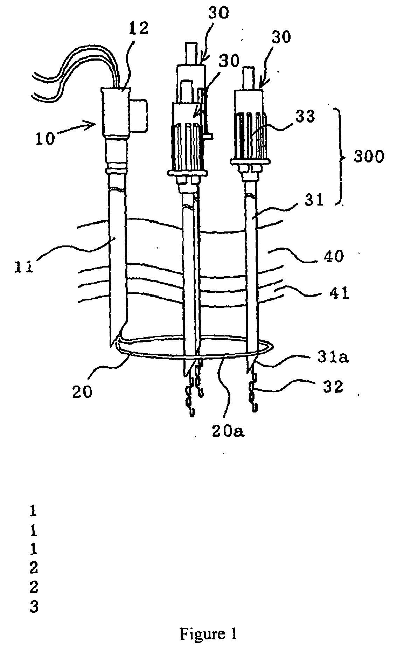

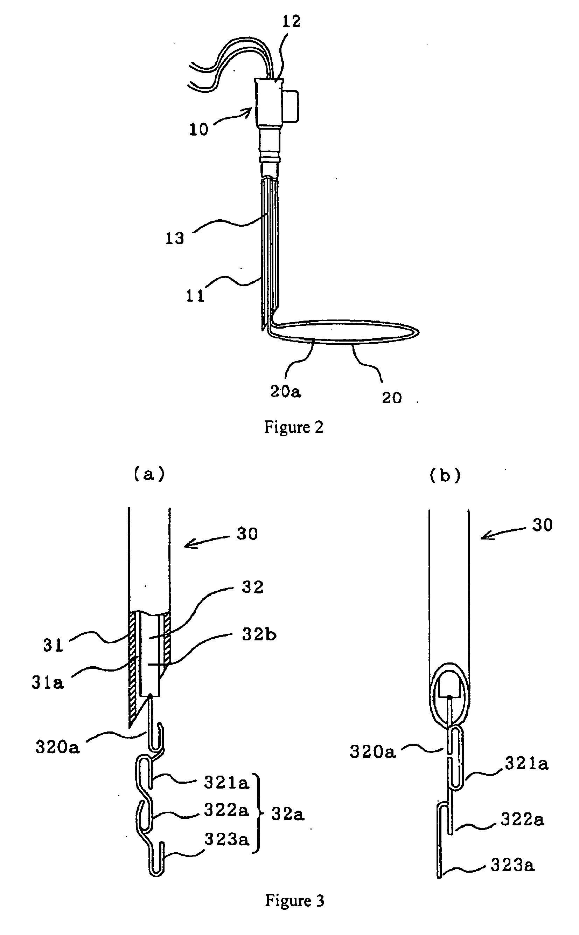

Figure 1 is a side view of the medical suturing device set pertaining to an embodiment of the present invention. Figure 2 is a partially axially cut-away mode side view of the suture and insertion puncture needle of Figure 1. Figure 3 is a side view of the essential elements of the extraction device of Figure 1. As shown in the figures, the medical suturing device set is structured by a insertion puncture needle 10 furnished with a suture 20, and several extraction devices 30 (3 in the illustrated example).

The insertion puncture needle 10 is structured using a puncture needle 11 and hub 12, with insertion through-hole 13 boring through these internally in the axial direction, such that suture 20 can slide within insertion through-hole 13. The puncture needle 11 is formed so that its apical tip has a sharp angle, and suture 20 protrudes from the apical tip so as to form a loop section 20a. The suture 20 may be constituted by a nylon thread, for example.

The extraction device 30 is formed by a extraction puncture needle 300, which has a hub 33 and a metal needle 31 having an apical tip formed into a sharp angle, and pulling out device 32. A cavity 31 a is formed axially within extraction puncture needle 300. The extraction device 30 is structured such that pulling out device 32 can slide within cavity 3 1 a and exit and enter through the apical tip of extraction puncture needle 300. The pulling out device 32 comprises a manipulation section 32b and an engagement member 32a that is contiguous with one end of the manipulation section 32b.

The engagement member 32a is an entity where multiple hook elements have been serially fixed in a sequential alignment (approximately linear): first hook element 320a, second hook element 321 a, third hook element 322a, and fourth hook element 323a, with an end of first hook element 320a being connected to one end of manipulation section 32b.

The respective hook elements constitute hook shapes shaped approximately like the letter "J," and are furnished with a base that can support part of suture 20, a primary extension that is connected to part of the base and extends towards the basal tip of pulling out device 32, a secondary extension that is connected to the other part of the base and that extends to the basal tip of pulling out device 32, and a gap that is formed so as to envelope the periphery of suture 20 by the base and the primary extension and the secondary extension. The linking of companion hook elements is formed by connections between the primary extension and secondary extension of adjacent hook elements.

In this case, the J-shaped flat surface of second hook element 321a is attached approximately perpendicular to the J-shaped flat surface of first hook element 320a, the J-shaped flat surface of third hook element 322a is attached approximately perpendicular to the J-shaped flat surface of second hook element 321 a, and the J-shaped flat surface of fourth hook element 323a is attached approximately perpendicular to the J-shaped flat surface of third hook element 322a. It is also possible for them to be connected to each other at any angle, not necessarily perpendicular. When the adjacent hook elements 320a, 321a, 322a, and 323a are positioned so as to be approximately perpendicular, the branches (projectional elements) of hook elements 320a, 321a, 322a, and 323a face towards the outside, extending in 4 directions.

The engagement member 32a may also be structured so that the J-shaped flat surface of first hook element 320a, the J-shaped flat surface of second hook element 321a, the J-shaped flat surface of third hook element 322a, and the J-shaped flat surface of fourth hook element 323a are positioned to be approximately on the same plane.

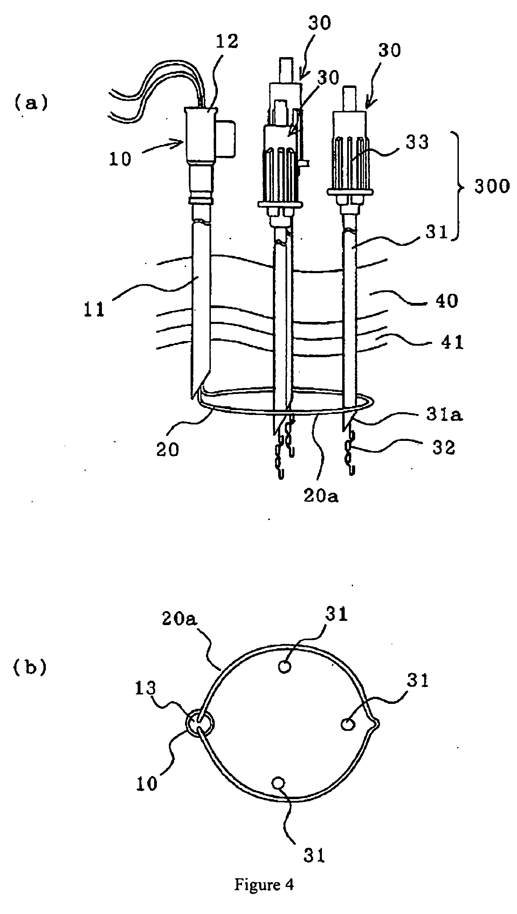

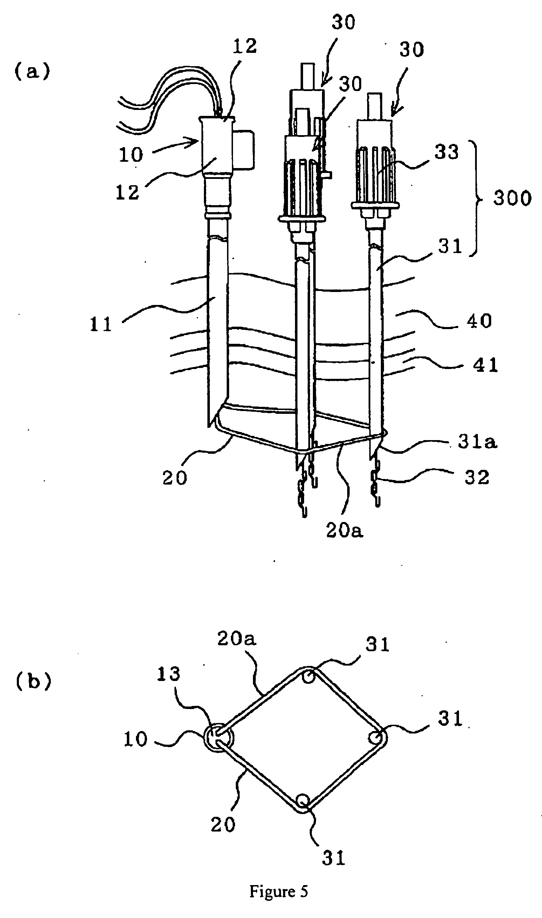

The method for using the medical suturing device set having the aforementioned structure (i.e., its operation), is explained in Figures 4-9. As shown in Figure 4, the hub 12 of insertion puncture needle 10 is grasped, and the gastric wall 41 is punctured from abdominal wall 40 by puncture needle 11. After the puncture, suture 20 is caused to slide along insertion through-hole 13 of insertion puncture needle 10, and suture 20 is caused to project from the apical tip of insertion through-hole 13, thus forming loop section 20a. Then there are multiple punctures of extraction device 30 within the loop section 20a formed by suture 20. The pulling out device 32 is thrust from the tip of metal needle 31 of extraction device 30, and suture 20 is drawn from the outside of hub 12 of insertion puncture needle 10. When this is done, as shown in Figure 5, loop section 20a is snugly attached to metal needle 31 of multiple extraction devices 30.

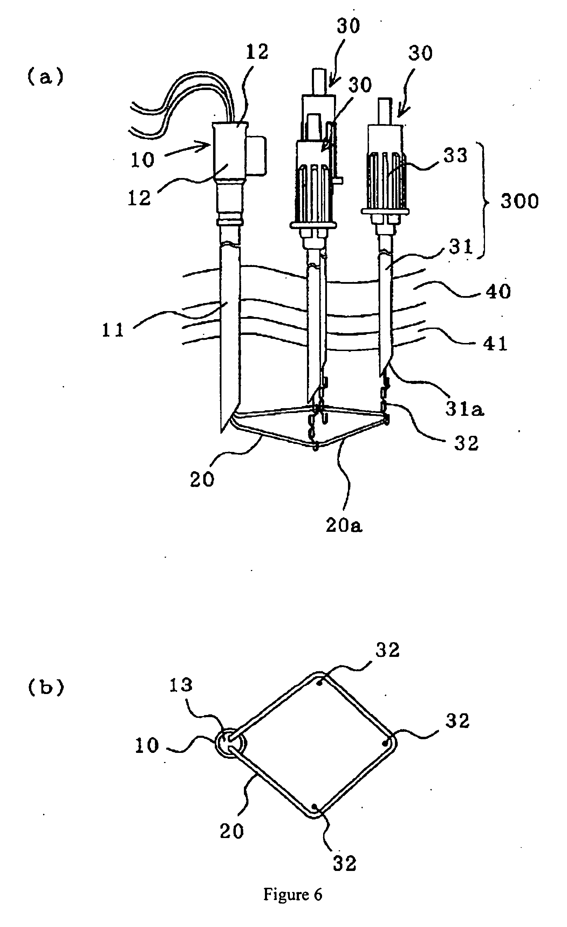

Next, as shown in Figure 6, extraction device 30 is slightly drawn up to engage the loop section 20a of suture 20 in engagement member 32a of pulling out device 32. Then, in the engaged condition, pulling out device 32 is stowed in cavity 31a of metal needle 31, and in this condition extraction device 30 is pulled upwards, and the upwardly pulled suture 20 is separated from extraction device 30. Then insertion puncture needle 10 is removed. When loop section 20a is pulled out by pulling out device 32, it may be removed from the organism without being temporarily stowed in the cavity of extraction puncture needle 300; pulling out device 32 may be removed from suture 20, and then extraction puncture needle 300 and insertion puncture needle 10 may be removed.

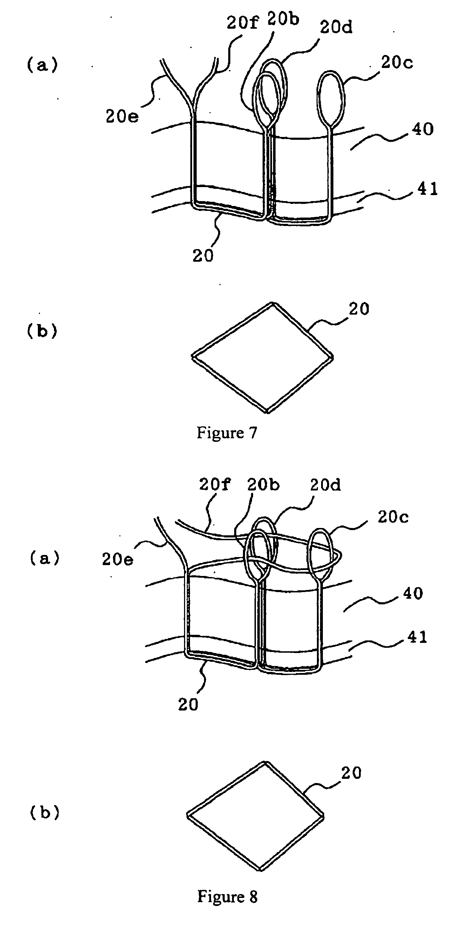

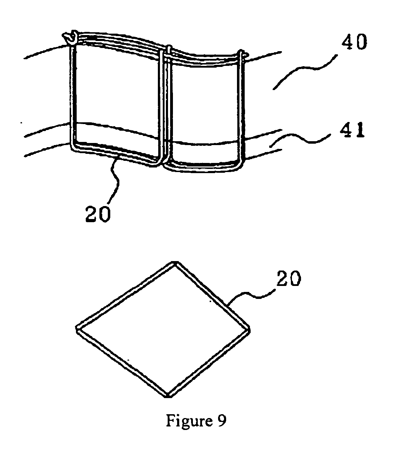

Then, as shown in Figure 7, a number of thread circles 20b, 20c, and 20d, equal to the number of puncturing extraction devices 30, are formed outside the body. Then, as shown in Figure 8, 20f, which is one of the suture ends positioned on the side of insertion puncture needle 10, is sequentially inserted into thread circles 20b, 20c, and 20d, which are positioned on the side of extraction device 30. Then, as shown in Figure 9, suture tips 20e and 20f are drawn together, knotted in the desired tightness, and thus fixed.

The foregoing explanation described a case where insertion puncture needle 10 was inserted and loop section 20a was formed, and extraction device 30 was inserted within loop section 20a, but it is also possible for extraction device 30 to first be inserted, then insert insertion puncture needle 10 and form loop section 20a, draw up insertion puncture needle 10, and position extraction device 30 within loop section 20a. The case was explained where the stomach was the organ to be sutured, but also applies to cases where other organs, such as the gallbladder or kidney, for example, are sutured.

In the embodiment disclosed, the structure is such that insertion puncture needle 10 and extraction device 30 are separated, so it is possible to pull out suture 20 formed in a ring shape through insertion through-hole 13 of insertion puncture needle 10 by means of pulling out device 32 of extraction device 30 at a position that is different from the insertion position, which makes it possible to freely select the puncture site together with the patient, and which also makes it possible to anchor the organ in plane shape, positioned within the organism, with only a single suture. Also, pulling out device 32 exits and enters along cavity 31a of metal needle 31 of extraction device 30, which makes it possible to reduce the invasiveness towards the body when suture 20 is drawn out of the organism. This makes it possible to securely anchor the desired organ to the paries with low invasiveness, even by inexperienced health care personnel.

## Claims

1. A surgical suture withdrawal device (30) for use in extracting a suture loop from within an organism to the outside of the organism, wherein the surgical suture withdrawal device (30) comprises:
a hollow needle (31) and
an engagement member (32) movable within the needle and capable of catching onto a suture positioned within the organism and drawing at least part of the suture outside of the organism,
**characterized in that** the engagement member has a form comprising multiple hook elements (320a, 321a, 322a, 323a).

2. The device according to claim 1, **characterized in that** the engagement member (32) may pass entirely through the hollow needle (31).

3. The device according to claim 1 or claim 2 wherein the engagement member comprises four hook elements (320a, 321a, 322a, 323a, 62a).

4. A surgical suturing set comprising one or more surgical suture withdrawal devices according to any of claims 1 to 3 and a suture loop insertion device (10) for introducing a suture loop into said organism.

5. The surgical suturing set according to claim 4 wherein the set comprises three suture withdrawal devices.

## Patentansprüche

1. Chirurgische Nahtrückzugsvorrichtung (30) zur Verwendung beim Ziehen einer Nahtschlaufe aus einem Organismus von innen nach außen, worin die chirurgische Nahtrückzugsvorrichtung (30) Folgendes umfasst: eine Hohlnadel (31) und ein Eingriffselement (32), das in der Nadel beweglich ist und auf einer Naht im Organismus eingreifen kann und zumindest einen Teil der Naht aus dem Organismus ziehen kann, **dadurch gekennzeichnet, dass** das Eingriffselement eine Form mit mehreren Hakenelementen (320a, 321a, 322a, 323a) aufweist.

2. Vorrichtung nach Anspruch 1, **dadurch gekennzeichnet, dass** das Eingriffselement (32) vollständig durch die Hohlnadel (31) geführt werden kann.

3. Vorrichtung nach Anspruch 1 oder 2, worin das Eingriffselement vier Hakenelemente (320a, 321a, 322a, 323a, 62a) umfasst.

4. Chirurgisches Nahtset mit einem oder mehreren chirurgischen Nahtrückzugsvorrichtungen nach einem der Ansprüche 1 bis 3 und einer Nahtschlaufeneinführungsvorrichtung (10) zum Einführen einer Nahtschlaufe in den Organismus.

5. Chirurgisches Nahtset nach Anspruch 4, worin das Set drei Nahtrückzugsvorrichtungen umfasst.

## Revendications

1. Dispositif (30) de retrait de suture chirurgicale destiné à être utilisé dans l'extraction d'une boucle de suture de l'intérieur d'un organisme vers l'extérieur de l'organisme, dans lequel le dispositif de retrait de suture chirurgicale (30) comporte :
une aiguille (31) creuse et
un élément (32) d'engagement mobile à l'intérieur de l'aiguille et pouvant s'accrocher à une suture positionnée dans l'organisme et tirer au moins une partie de la suture hors de l'organisme,
**caractérisé en ce que** l'élément d'engagement a une forme constituée de multiples crochets (320a, 321a, 322a, 323a).

2. Dispositif selon la revendication 1, **caractérisé en ce que** l'élément (32) d'engagement peut traverser entièrement l'aiguille (31) creuse.

3. Dispositif selon la revendication 1 ou la revendication 2 dans lequel l'élément d'engagement comporte quatre crochets (320a, 321a, 322a, 323a, 62a).

4. Ensemble de suture chirurgicale comportant un ou plusieurs dispositifs de retrait de suture chirurgicale selon l'une quelconque des revendications 1 à 3 et un dispositif d'introduction d'une boucle de suture (10) pour introduire une boucle de suture dans ledit organisme.

5. Ensemble de suture chirurgicale selon la revendication 4 dans lequel l'ensemble comporte trois dispositifs de retrait de suture.
